# EUROPEAN PATENT SPECIFICATION

(11) **EP 1 280 571 B1**
(45) Date of publication and mention of the grant of the patent: **26.10.2005**
(21) Application number: 01935490.1
(22) Date of filing: 14.05.2001
(51) Int. Cl.: A61L 31/16

(54) **DELIVERY DEVICES FOR TREATMENT OF VASCULAR DISEASE**
WIRKSTOFFABGABESYSTEME ZUR BEHANDLUNG VON GEFÄSSERKRANKUNGEN
DISPOSITIFS D'ADMINISTRATION POUR LE TRAITEMENT DES MALADIES VASCULAIRES

(30) Priority: 12.05.2000 US 204417 P; 19.05.2000 US 575480; 07.05.2001 US 850485
(43) Date of publication of application: 05.02.2003
(73) Proprietor: Cordis Corporation, Miami Lakes, Florida 33014 (US)
(72) Inventor: FALOTICO, Robert, Belle Mead, NJ 08502 (US); KOPIA, Gregory, A., Hillsborough, NJ 08844 (US); LANDAU, George, Verona, NJ 07044 (US); LLANOS, Gerard, H., Stewartsville, NJ 08886 (US); NARAYANAN, Pallassana, V., Belle Mead, NJ 08502 (US); PAPANDREOU, George, Kendall Park, NJ 08824 (US)
(74) Representative: Belcher, Simon James
(86) International application number: PCT/US2001/015562
(87) International publication number: WO 2001/087375

(56) References cited:
- EP-A- 0 568 310
- EP-A- 0 950 386
- WO-A-00/27445
- WO-A-98/36784

## Description

### BACKGROUND OF THE INVENTION

### 1. Field of the Invention

The present invention relates to the administration of drug combinations for the prevention and treatment of vascular disease, and more particularly to an intraluminal medical device for the local delivery of drug combinations for the prevention and treatment of vascular disease caused by injury.

### 2. Discussion of the Related Art

Many individuals suffer from circulatory disease caused by a progressive blockage of the blood vessels that perfuse the heart and other major organs with nutrients. More severe blockage of blood vessels in such individuals often leads to hypertension, ischemic injury, stroke, or myocardial infarction. Atherosclerotic lesions, which limit or obstruct coronary blood flow, are the major cause of ischemic heart disease. Percutaneous transluminal coronary angioplasty is a medical procedure whose purpose is to increase blood flow through an artery. Percutaneous transluminal coronary angioplasty is the predominant treatment for coronary vessel stenosis. The increasing use of this procedure is attributable to its relatively high success rate and its minimal invasiveness compared with coronary bypass surgery. A limitation associated with percutaneous transluminal coronary angioplasty is the abrupt closure of the vessel which may occur immediately after the procedure and restenosis which occurs gradually following the procedure. Additionally, restenosis is a chronic problem in patients who have undergone saphenous vein bypass grafting. The mechanism of acute occlusion appears to involve several factors and may result from vascular recoil with resultant closure of the artery and/or deposition of blood platelets and fibrin along the damaged length of the newly opened blood vessel.

Restenosis after percutaneous transluminal coronary angioplasty is a more gradual process initiated by vascular injury. Multiple processes, including thrombosis, inflammation, growth factor and cytokine release, cell proliferation; cell migration and extracellular matrix synthesis each contribute to the restenotic process.

While the exact mechanism of restenosis is not completely understood, the general aspects of the restenosis process have been identified. In the normal arterial wall, smooth muscle cells proliferate at a low rate, approximately less than 0.1 percent per day. Smooth muscle cells in the vessel walls exist in a contractile phenotype characterized by eighty to ninety percent of the cell cytoplasmic volume occupied with the contractile apparatus. Endoplasmic reticulum, Golgi, and free ribosomes are few and are located in the perinuclear region. Extracellular matrix surrounds the smooth muscle cells and is rich in heparin-like glycosylaminoglycans which are believed to be responsible for maintaining smooth muscle cells in the contractile phenotypic state (Campbell and Campbell, 1985).

Upon pressure expansion of an intracoronary balloon catheter during angioplasty, smooth muscle cells within the vessel wall become injured, initiating a thrombotic and inflammatory response. Cell derived growth factors such as platelet derived growth factor, fibroblast growth factor, epidermal growth factor, thrombin, etc., released from platelets, invading macrophages and/or leukocytes, or directly from the smooth muscle cells provoke proliferative and migratory responses in medial smooth muscle cells. These cells undergo a change from the contractile phenotype to a synthetic phenotype characterized by only a few contractile filament bundles, extensive rough endoplasmic reticulum, Golgi and free ribosomes. Proliferation/migration usually begins within one to two days post-injury and peaks several days thereafter (Campbell and Campbell, 1987; Clowes and Schwartz, 1985).

Daughter cells migrate to the intimal layer of arterial smooth muscle and continue to proliferate and secrete significant amounts of extracellular matrix proteins. Proliferation, migration and extracellular matrix synthesis continue until the damaged endothelial layer is repaired at which time proliferation slows within the intima, usually within seven to fourteen days post-injury. The newly formed tissue is called neointima. The further vascular narrowing that occurs over the next three to six months is due primarily to negative or constrictive remodeling.

Simultaneous with local proliferation and migration, inflammatory cells invade the site of vascular injury. Within three to seven days post-injury, inflammatory cells have migrated to the deeper layers of the vessel wall. In animal models employing either balloon injury or stent implantation, inflammatory cells may persist at the site of vascular injury for at least thirty days (Tanaka et al., 1993; Edelman et al., 1998). Inflammatory cells therefore are present and may contribute to both the acute and chronic phases of restenosis.

Numerous agents have been examined for presumed anti-proliferative actions in restenosis and have shown some activity in experimental animal models. Some of the agents which have been shown to successfully reduce the extent of intimal hyperplasia in animal models include: heparin and heparin fragments (Clowes, A.W. and Karnovsky M., Nature 265: 25-26, 1977; Guyton, J.R. et al., Circ. Res., 46: 625-634, 1980; Clowes, A.W. and Clowes, M.M., Lab. Invest. 52: 611-616, 1985; Clowes, A.W. and Clowes, M.M., Circ. Res. 58: 839-845, 1986; Majesky et al., Circ. Res. 61: 296-300, 1987; Snow et al., Am. J. Pathol. 137: 313-330, 1990; Okada, T. et al., Neurosurgery 25: 92-98, 1989), colchicine (Currier, J.W. et al., Circ. 80: 11-66, 1989), taxol (Sollot, S.J. et al., J. Clin. Invest. 95: 1869-1876, 1995), angiotensin converting enzyme (ACE) inhibitors (Powell, J.S. et al., Science, 245: 186-188, 1989), angiopeptin (Lundergan, C.F. et al. Am. J. Cardiol. 17(Suppl. B):132B-136B, 1991), cyclosporin A (Jonasson, L. et al., Proc. Natl., Acad. Sci., 85: 2303, 1988), goat-anti-rabbit PDGF antibody (Ferns, G.A.A., et al., Science 253: 1129-1132, 1991), terbinafine (Nemecek, G.M. et al., J. Pharmacol. Exp. Thera. 248: 1167-1174, 1989), trapidil (Liu, M.W. et al., Circ. 81: 1089-1093, 1990), tranilast (Fukuyama, J. et al., Eur. J. Pharmacol. 318: 327-332, 1996), interferon-gamma (Hansson, G.K. and Holm, J., Circ. 84: 1266-1272,1991), rapamycin (Marx, S.O. et al., Circ. Res. 76: 412-417, 1995), corticosteroids (Colburn, M.D. et al., J. Vasc. Surg. 15: 510-518, 1992), see also Berk, B.C. et al., J. Am. Coll. Cardiol. 17: 111 B-117B, 1991), ionizing radiation (Weinberger, J. et al., Int. J. Rad. Onc. Biol. Phys. 36: 767-775, 1996), fusion toxins (Farb, A. et al., Circ. Res. 80: 542-550, 1997) antisense oligonucleotides (Simons, M. et al., Nature 359: 67-70, 1992) and gene vectors (Chang, M.W. et al., J. Clin. Invest. 96: 2260-2268, 1995). Anti-proliferative effects on smooth muscle cells *in vitro* have been demonstrated for many of these agents, including heparin and heparin conjugates, taxol, tranilast, colchicine, ACE inhibitors, fusion toxins, antisense oligonucleotides, rapamycin and ionizing radiation. Thus, agents with diverse mechanisms of smooth muscle cell inhibition may have therapeutic utility in reducing intimal hyperplasia.

However, in contrast to animal models, attempts in human angioplasty patients to prevent restenosis by systemic pharmacologic means have thus far been unsuccessful. Neither aspirin-dipyridamole, ticlopidine, anti-coagulant therapy (acute heparin, chronic warfarin, hirudin or hirulog), thromboxane receptor antagonism nor steroids have been effective in preventing restenosis, although platelet inhibitors have been effective in preventing acute reocclusion after angioplasty (Mak and Topol, 1997; Lang et al., 1991; Popma et al., 1991). The platelet GP IIb/IIIa receptor, antagonist, Reopro is still under study but has not shown promising results for the reduction in restenosis following angioplasty and stenting. Other agents, which have also been unsuccessful in the prevention of restenosis, include the calcium channel antagonists, prostacyclin mimetics, angiotensin converting enzyme inhibitors, serotonin receptor antagonists, and anti-proliferative agents. These agents must be given systemically, however, and attainment of a therapeutically effective dose may not be possible; anti-proliferative (or anti-restenosis) concentrations may exceed the known toxic concentrations of these agents so that levels sufficient to produce smooth muscle inhibition may not be reached (Mak and Topol, 1997; Lang et al., 1991; Popma et al., 1991).

Additional clinical trials in which the effectiveness for preventing restenosis utilizing dietary fish oil supplements or cholesterol lowering agents has been examined showing either conflicting or negative results so that no pharmacological agents are as yet clinically available to prevent post-angioplasty restenosis (Mak and Topol, 1997; Franklin and Faxon, 1993: Serruys, P.W. et al., 1993). Recent observations suggest that the antilipid/antioxidant agent, probucol may be useful in preventing restenosis but this work requires confirmation (Tardif et al., 1997; Yokoi, et al., 1997). Probucol is presently not approved for use in the United States and a thirty-day pretreatment period would preclude its use in emergency angioplasty. Additionally, the application of ionizing radiation has shown significant promise in reducing or preventing restenosis after angioplasty in patients with stents (Teirstein et al., 1997). Currently, however, the most effective treatments for restenosis are repeat angioplasty, atherectomy or coronary artery bypass grafting, because no therapeutic agents currently have Food and Drug Administration approval for use for the prevention of post-angioplasty restenosis.

Unlike systemic pharmacologic therapy, stents have proven effective in significantly reducing restenosis. Typically, stents are balloon-expandable slotted metal tubes (usually, but not limited to, stainless steel), which, when expanded within the lumen of an angioplastied coronary artery, provide structural support through rigid scaffolding to the arterial wall. This support is helpful in maintaining vessel lumen patency. In two randomized clinical trials, stents increased angiographic success after percutaneous transluminal coronary angioplasty, by increasing minimal lumen diameter and reducing, but not eliminating, the incidence of restenosis at six months (Serruys et al., 1994; Fischman et al., 1994).

Additionally, the heparin coating of stents appears to have the added benefit of producing a reduction in sub-acute thrombosis after stent implantation (Serruys etal., 1996). Thus, sustained mechanical expansion of a stenosed coronary artery with a stent has been shown to provide some measure of restenosis prevention, and the coating of stents with heparin has demonstrated both the feasibility and the clinical usefulness of delivering drugs locally, at the site of injured tissue.

WO-98/36784 discloses an implantable medical device including a structure adapted for introduction into a patient, at least one coating layer posited on one surface of the structure, and at least one layer of a first bioactive material posited over at least a portion of the at least one coating layer wherein said at least one coating layer provides for a controlled release of the bioactive material from the at least one coating layer. However, if the device comprises a second bioactive material layer, in which the second bioactive material can be, but need not be, different from the first bioactive material of the first bioactive material layer, these layers can not be posited on the same surface of the device without the intermediate porous layer.

EP-950386 provides a stent with local rapamycin delivery. Rapamycin can be applied as a polymer/drug mixture and can be modified to contain a hydrolytically or enzymatically labile covalent bond for attaching to the surface of the stent. The stent can additionally comprise reservoirs of drugs.

Accordingly, there exists a need for effective drugs and drug delivery systems for the effective prevention and treatment of neointimal thickening that occurs after percutaneous transluminal coronary angioplasty and stent implantation.

### SUMMARY OF THE INVENTION

The drug combinations and delivery devices of the present invention provide a means for overcoming the difficulties associated with the methods and devices currently in use as briefly described above.

The present invention is directed to an intraluminal medical device. The intraluminal medical device comprises a stent having a substantially tubular structure, the tubular structure having an inner surface and an outer surface, a layer of one or more anti-proliferative compounds affixed to the outer surface of the tubular structure, a first layer of one or more anti-coagulant compounds affixed to the inner surface of the tubular structure, and a second layer of one or more anti-coagulant compounds affixed to the layer of one or more anti-proliferative compounds affixed to the outer surface of the tubular structure.

In accordance with another aspect, the present invention is directed to an intraluminal medical device. The intraluminal medical device comprises a stent having a plurality of bands, the bands being expansible within the lumen of the body, and at least one of the bands including at least one reservoir in an inner and outer surface of the bands, a therapeutic dosage of one or more anti-proliferative compounds immobilized in at least one reservoir in the outer surface of the bands, and a therapeutic dosage of one or more anti-coagulant compounds immobilized in at least one reservoir in the inner surface of the bands.

In accordance with another aspect, the present invention is directed to a method for the treatment of injury in vessel walls. The method comprises the local delivery of combinations of at least two agents to a patient in therapeutic dosage amounts.

The intraluminal medical device of the present invention utilizes one or more drugs, agents or compounds for the prevention and treatment of vascular disease caused by injury. An intraluminal medical device, for example, a stent may be coated with one or more drugs, agents or compounds that reduce smooth muscle cell proliferation, reduce inflammation and reduce thrombosis. Essentially, stents or other similar medical devices, e.g. grafts, in combination with one or more drugs, agents or compounds which prevent or reduce smooth muscle cell proliferation, reduce thrombosis and reduce inflammation may provide the most efficacious treatment of restenosis and other vascular tissue injury/disease. The local administration of these drugs, agents or compounds will result in higher vessel tissue concentrations and lower toxicity due to reduced dosages than that associated with systemic delivery of the same drugs, agents or compounds.

The intraluminal medical device of the present invention may be selectively coated with the drugs, agents or compounds such that the most efficient delivery of the drugs, agents or compounds may be achieved. For example, the drugs, agents or compounds for preventing or reducing smooth muscle cell proliferation may be incorporated into the device on the surface which comes in direct contact with the affected tissue while the drugs, agents or compounds for inhibiting coagulation may be incorporated into the device on the surface which comes into contact with the blood.

The intraluminal medical device of the present invention makes use of various techniques and methodologies of affixing therapeutic drugs, agents or compounds to intraluminal medical devices. Accordingly, delivery of these drugs, agents or compounds may be optimally achieved. Since the drugs, agents or compounds are locally delivered, the patient, as well as the physician, will not have to be concerned with the need for continuous administration, e.g. orally or intravenously.

### BRIEF DESCRIPTION OF THE DRAWINGS

The foregoing and other features and advantages of the invention will be apparent from the following, more particular description of preferred embodiments of the invention, as illustrated in the accompanying drawings.
Figure 1 is a view along the length of a stent (ends not shown) prior to expansion showing the exterior surface of the stent and the characteristic banding pattern.
Figure 2 is a perspective view of the stent of Figure 1 having reservoirs in accordance with the present invention.
Figure 4 is a cross-sectional view of a band of the stent of Figure 1 having drug coatings thereon in accordance with an embodiment of the present invention.
Figure 5 is a cross-sectional view of a band of the stent of Figure 1 having drug coatings thereon in accordance with an embodiment of the present invention.

### DETAILED DESCRIPTION OF THE PREFERRED EMBODIMENTS

The drug combinations and delivery devices of the present invention may be utilized to effectively prevent and treat vascular disease, and in particular, vascular disease caused by injury. Various medical treatment devices utilized in the treatment of vascular disease may ultimately induce further complications. For example, balloon angioplasty is a procedure utilized to increase blood flow through an artery and is the predominant treatment for coronary vessel stenosis. However, as stated above, the procedure typically causes a certain degree of damage to the vessel wall, thereby potentially exacerbating the problem at a point later in time. Although other procedures and diseases may cause similar injury, the present invention will be described with respect to the treatment of restenosis and related complications following percutaneous transluminal coronary angioplasty.

As stated previously, the implantation of a coronary stent in conjunction with balloon angioplasty is highly effective in treating acute vessel closure and may reduce the risk of restenosis. Intravascular ultrasound studies (Mintz et al., 1996) suggest that coronary stenting effectively prevents vessel constriction and that most of the late luminal loss after stent implantation is due to plaque growth, probably related to neointimal hyperplasia. The late luminal loss after coronary stenting is almost two times higher than that observed after conventional balloon angioplasty. Thus, inasmuch as stents prevent at least a portion of the restenosis process, a combination of drugs, agents or compounds, which prevents smooth muscle cell proliferation, reduces inflammation and reduces coagulation or prevents smooth muscle cell proliferation by multiple mechanisms, reduces inflammation and reduces coagulation combined with a stent may provide the most efficacious treatment for post-angioplasty restenosis. The systemic use of drugs, agents or compounds in combination with the local delivery of the same or different drugs, agents or compounds may also provide a beneficial treatment option.

The local delivery of multiple drugs, agents or compounds from a stent has the following advantages; namely, the prevention of vessel recoil and remodeling through the scaffolding action of the stent and the prevention of multiple components of neointimal hyperplasia or restenosis as well as a reduction in inflammation and thrombosis. This local administration of drugs, agents or compounds to stented coronary arteries may also have additional therapeutic benefit. For example, higher tissue concentrations of the drugs, agents, or compounds can be achieved utilizing local delivery, rather than systemic administration. In addition, reduced systemic toxicity may be achieved utilizing local delivery rather than systemic administration while maintaining higher tissue concentrations. Also in utilizing local delivery from a stent rather than systemic administration, a single procedure may suffice with better patient compliance. An additional benefit of combination drug/agent/compound therapy may be to reduce the dose of each of the therapeutic drugs, agents or compounds, thereby limiting their toxicity, while still achieving a reduction in restenosis, inflammation and thrombosis. Local stent-based therapy is therefore a means of improving the therapeutic ratio (efficacy/toxicity) of anti-restenosis, anti-inflammatory, anti-thrombotic drugs, agents or compounds.

There are a multiplicity of stent designs that may be utilized following percutaneous transluminal coronary angioplasty. Although any number of stent designs may be utilized in accordance with the present invention, for simplicity, one particular stent will be described in exemplary embodiments of the present invention. The skilled artisan will recognize that any number of stents may be utilized in connection with the present invention.

A stent is commonly used as a tubular structure left inside the lumen of a duct to relieve an obstruction. Commonly, stents are inserted into the lumen in a non-expanded form and are then expanded autonomously, or with the aid of a second device *in situ.* A typical method of expansion occurs through the use of a catheter-mounted angioplasty balloon which is inflated within the stenosed vessel or body passageway in order to shear and disrupt the obstructions associated with the wall components of the vessel and to obtain an enlarged lumen.

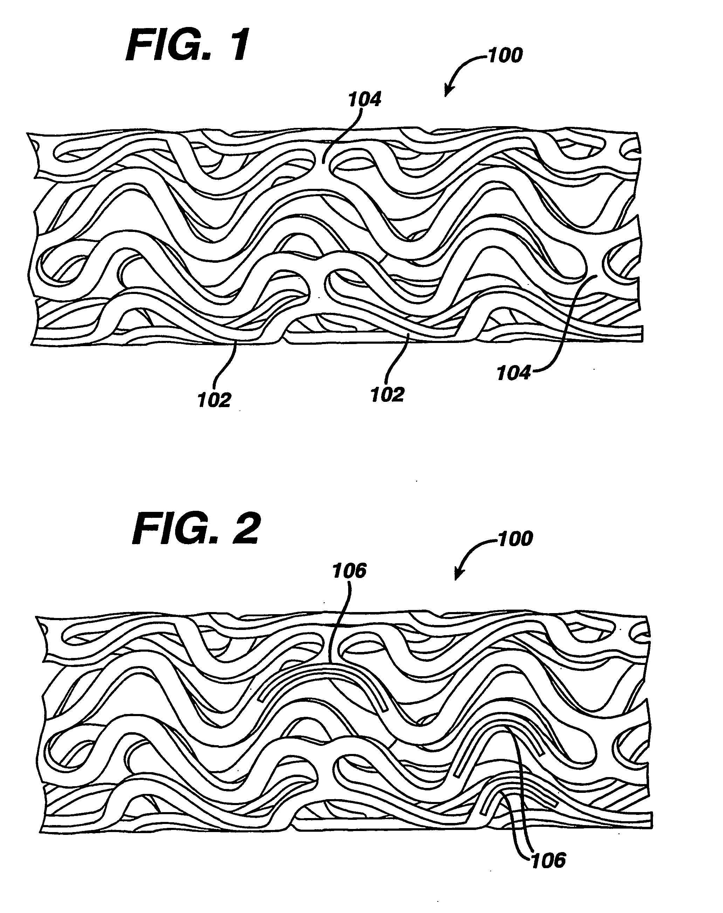

Figure 1 illustrates an exemplary stent 100 which may be utilized in accordance with an exemplary embodiment of the present invention. The expandable cylindrical stent 100 comprises a fenestrated structure for placement in a blood vessel, duct or lumen to hold the vessel, duct or lumen open, more particularly for protecting a segment of artery from restenosis after angioplasty. The stent 100 may be expanded circumferentially and maintained in an expanded configuration, that is circumferentially or radially rigid. The stent 100 is axially flexible and when flexed at a band, the stent 100 avoids any externally-protruding component parts.

The stent 100 generally comprises first and second ends with an intermediate section therebetween. The stent 100 has a longitudinal axis and comprises a plurality of longitudinally disposed bands 102, wherein each band 102 defines a generally continuous wave along a line segment parallel to the longitudinal axis. A plurality of circumferentially arranged links 104 maintain the bands 102 in a substantially tubular structure. Essentially, each longitudinally disposed band 102 is connected at a plurality of periodic locations, by a short circumferentially arranged link 104 to an adjacent band 102. The wave associated with each of the bands 102 has approximately the same fundamental spatial frequency in the intermediate section, and the bands 102 are so disposed that the wave associated with them are generally aligned so as to be generally in phase with one another. As illustrated in the figure, each longitudinally arranged band 102 undulates through approximately two cycles before there is a link to an adjacent band 102.

The stent 100 may be fabricated utilizing any number of methods. For example, the stent 100 may be fabricated from a hollow or formed stainless steel tube that may be machined using lasers, electric discharge milling, chemical etching or other means. The stent 100 is inserted into the body and placed at the desired site in an unexpanded form. In one embodiment, expansion may be effected in a blood vessel by a balloon catheter, where the final diameter of the stent 100 is a function of the diameter of the balloon catheter used.

It should be appreciated that a stent 100 in accordance with the present invention may be embodied in a shape-memory material, including, for example, an appropriate alloy of nickel and titanium or stainless steel. In this embodiment after the stent 100 has been formed it may be compressed so as to occupy a space sufficiently small as to permit its insertion in a blood vessel or other tissue by insertion means, wherein the insertion means include a suitable catheter, or flexible rod. On emerging from the catheter, the stent 100 may be configured to expand into the desired configuration where the expansion is automatic or triggered by a change in pressure, temperature or electrical stimulation.

Figure 2 illustrates an exemplary embodiment of the present invention utilizing the stent 100 illustrated in Figure 1. As illustrated, the stent 100 may be modified to comprise one or more reservoirs 106. Each of the reservoirs 106 may be opened or closed as desired. These reservoirs 106 may be specifically designed to hold the drugs, agents or compounds to be delivered. Regardless of the design of the stent 100, it is preferable to have the drugs, agents or compounds dosage applied with enough specificity and a sufficient concentration to provide an effective dosage in the lesion area. In this regard, the reservoir size in the bands 102 is preferably sized to adequately apply the drugs, agents or compounds dosage at the desired location and in the desired amount.

In an alternate exemplary embodiment, the entire inner and outer surface of the stent 100 may be coated with various drug, agent or compound combinations in therapeutic dosage amounts. A detailed description of various drugs, agents, or compounds as well as exemplary coating techniques is described below. It is, however, important to note that the coating techniques may vary depending on the drugs, agents or compounds. Also, the coating techniques may vary depending on the material forming the stent or other intraluminal medical device.

Rapamycin is a macroyclic triene antibiotic produced by streptomyces hygroscopicus as disclosed in U. S. Patent No. 3,929,992. It has been found that rapamycin among other things inhibits the proliferation of vascular smooth muscle cells in vivo. Accordingly, rapamycin may be utilized in treating intimal smooth muscle cell hyperplasia, restenosis, and vascular occlusion in a mammal, particularly following either biologically or mechanically mediated vascular injury, or under conditions that would predispose a mammal to suffering such a vascular injury. Rapamycin functions to inhibit smooth muscle cell proliferation and does not interfere with the re-endothelialization of the vessel walls.

Rapamycin reduces vascular hyperplasia by antagonizing smooth muscle proliferation in response to mitogenic signals that are released during an angioplasty. Inhibition of growth factor and cytokine mediated smooth muscle proliferation at the lateGI phase of the cell cycle is believed to be the dominant mechanism of action of rapamycin. However, rapamycin is also known to prevent T-cell proliferation and differentiation when administered systemically. This is the basis for its immunosuppresive activity and its ability to prevent graft rejection.

Although the anti-proliferative effects of rapamycin may be achieved through systemic use, superior results may be achieved through the local delivery of the compound. Essentially, rapamycin is effective in the tissues, which are in proximity to the compound, and has diminished effect as the distance from the delivery device increases. In order to take advantage of this effect, one would want rapamycin to be in direct contact with the lumen walls.
Accordingly, in a preferred embodiment, rapamycin is incorporated into the outer surface of the stent or portions thereof. Essentially, the rapamycin is preferably incorporated into the stent 100, illustrated in Figure 1, where the stent 100 makes contact with the lumen wall.

Rapamycin may be incorporated into or affixed to the stent in a number of ways. In the exemplary embodiment, the rapamycin is directly incorporated into a polymeric matrix and sprayed onto the outer surface of the stent. The rapamycin elutes from the polymeric matrix over time and enters the surrounding tissue. The rapamycin preferably remains on the stent for at least three days up to approximately six months, and more preferably between seven and thirty days.

Any number of non-erodible polymers may be utilized in conjunction with the rapamycin. In the preferred embodiment, the polymeric matrix comprises two layers. The base layer comprises a solution of ethylene-co-vinylacetate and polybutylmethacrylate. The rapamycin is incorporated into this base layer. The outer layer comprises only polybutylmethacrylate and acts as a diffusion barrier to prevent the rapamycin from eluting too quickly. The thickness of the outer layer or top coat determines the rate at which the rapamycin elutes from the matrix. Essentially, the rapamycin elutes from the matrix by diffusion through the polymer molecules. Polymers are permeable, thereby allowing solids, liquids and gases to escape therefrom. The total thickness of the polymeric matrix is in the range from about 1 micron to about 20 microns or greater.

The ethylene-co-vinylacetate, polybutylmethacrylate and rapamycin solution may be incorporated into or onto the stent in a number of ways. For example, the solution may be sprayed onto the stent or the stent may be dipped into the solution. In one exemplary embodiment, the solution is sprayed onto the stent and then allowed to dry. In another exemplary embodiment, the solution may be electrically charged to one polarity and the stent electrically changed to the opposite polarity. In this manner, the solution and stent will be attracted to one another. In using this type of spraying process, waste may be reduced and more precise control over the thickness of the coat may be achieved.

Since rapamycin acts by entering the surrounding tissue, it is preferably only affixed to the surface of the stent making contact with one tissue. Typically, only the outer surface of the stent makes contact with the tissue. Accordingly, in a preferred embodiment, only the outer surface of the stent is coated with rapamycin.

The circulatory system, under normal conditions, has to be self-sealing, otherwise continued blood loss from an injury would be life threatening. Typically, all but the most catastrophic bleeding is rapidly stopped though a process known as hemostasis. Hemostasis occurs through a progression of steps. At high rates of flow, hemostasis is a combination of events involving platelet aggregation and fibrin formation. Platelet aggregation leads to a reduction in the blood flow due to the formation of a cellular plug while a cascade of biochemical steps leads to the formation of a fibrin clot.

Fibrin clots, as stated above, form in response to injury. There are certain circumstances where blood clotting or clotting in a specific area may pose a health risk. For example, during percutaneous transluminal coronary angioplasty, the endothelial cells of the arterial walls are typically injured, thereby exposing the sub-endothelial cells. Platelets adhere to these exposed cells. The aggregating platelets and the damaged tissue initiate further biochemical process resulting in blood coagulation. Platelet and fibrin blood clots may prevent the normal flow of blood to critical areas. Accordingly, there is a need to control blood clotting in various medical procedures. Compounds that do not allow blood to clot are called anti-coagulants. Essentially, an anti-coagulant is an inhibitor of thrombin formation or function. These compounds include drugs such as heparin and hirudin. As used herein, heparin includes all direct or indirect inhibitors of thrombin or Factor Xa.

In addition to being an effective anti-coagulant, heparin has also been demonstrated to inhibit smooth muscle cell growth *in vivo.* Thus, heparin may be effectively utilized in conjunction with rapamycin in the treatment of vascular disease. Essentially, the combination of rapamycin and heparin may inhibit smooth muscle cell growth via two different mechanisms in addition to the heparin acting as an anti-coagulant.

Because of its multifunctional chemistry, heparin may be immobilized or affixed to a stent in a number of ways. For example, heparin may be immobilized onto a variety of surfaces by various methods, including the photolink methods set forth in U.S. Patent Nos. 3,959,078 and 4,722,906 to Guire et al. and U.S. Patent Nos. 5,229,172; 5,308,641; 5,350,800 and 5,415,938 to Cahalan et al. Heparinized surfaces have also been achieved by controlled release from a polymer matrix, for example, silicone rubber, as set forth in U.S. Patent Nos. 5,837,313; 6,099,562 and 6,120,536 to Ding et al.

In one exemplary embodiment, heparin may be immobilized onto the stent as briefly described below. The surface onto which the heparin is to be affixed is cleaned with ammonium peroxidisulfate. Once cleaned, alternating layers of polyethylenimine and dextran sulfate are deposited thereon. Preferably, four layers of the polyethylenimine and dextran sulfate are deposited with a final layer of polyethylenimine. Aldehyde-end terminated heparin is then immobilized to this final layer and stabilized with sodium cyanoborohydride. This process is set forth in U.S. Patent Nos. 4,613,665; 4,810,784 to Larm and 5,049,403 to Larm et al.

Unlike rapamycin, heparin acts on circulating proteins in the blood and heparin need only make contact with blood to be effective.

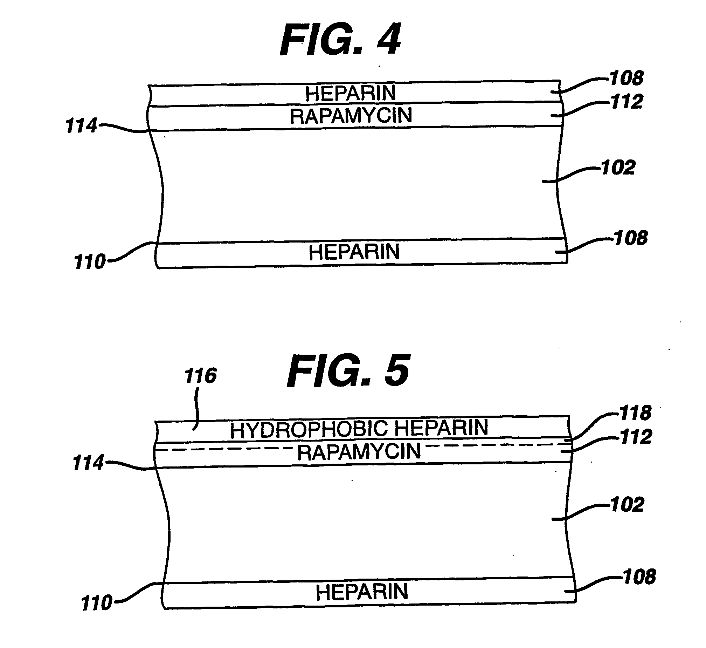

The stent may comprise a heparin layer immobilized on its inner surface, and rapamycin and heparin on its outer surface. Utilizing current coating techniques, heparin tends to form a stronger bond with the surface it is immobilized to then does rapamycin. Accordingly, it may be possible to first immobilize the rapamycin to the outer surface of the stent and then immobilize a layer of heparin to the rapamycin layer. In this embodiment, the rapamycin may be more securely affixed to the stent while still effectively eluting from its polymeric matrix, through the heparin and into the surrounding tissue. Figure 4 illustrates a cross-section of a band 102 of the stent 100 illustrated in Figure 1. As illustrated, the band 102 is coated with heparin 108 on its inner surface 110 and with rapamycin 112 and heparin 108 on its outer surface 114.

There are a number of possible ways to immobilize, i.e., entrapment or covalent linkage with an erodible bond, the heparin layer to the rapamycin layer. For example, heparin may be introduced into the top layer of the polymeric matrix. In other embodiments, different forms of heparin may be directly immobilized onto the top coat of the polymeric matrix, for example, as illustrated in Figure 5. As illustrated, a hydrophobic heparin layer 116 may be immobilized onto the top coat layer 118 of the rapamycin layer 112. A hydrophobic form of heparin is utilized because rapamycin and heparin coatings represent incompatible coating application technologies. Rapamycin is an organic solvent-based coating and heparin is a water-based coating.

As stated above, a rapamycin coating may be applied to stents by a dip, spray or spin coating method, and/or any combination of these methods. Various polymers may be utilized. For example, as described above, polyethylene-co-vinyl acetate and polybutyl methacrylate blends may be utilized. Other polymers may also be utilized, but not limited to, for example, polyvinylidene fluoride-co-hexafluoropropylene and polyethylbutyl methacrylate-co-hexyl methacrylate. Also as described above, barrier or top coatings may also be applied to modulate the dissolution of rapamycin from the polymer matrix. In the exemplary embodiment described above, a thin layer of heparin is applied to the surface of the polymeric matrix. Because these polymer systems are hydrophobic and incompatible with the hydrophilic heparin, appropriate surface modifications may be required.

The application of heparin to the surface of the polymeric matrix may be performed in various ways and utilizing various biocompatible materials. For example, in one embodiment, in water or alcoholic solutions, polyethylene imine may be applied on the stents, with care not to degrade the rapamycin (e.g., pH < 7, low temperature), followed by the application of sodium heparinate in aqueous or alcoholic solutions. As an extension of this surface modification, covalent heparin may be linked on polyethylene imine using amide-type chemistry (using a carbondiimide activator, e.g. EDC) or reductive amination chemistry (using CBAS-heparin and sodium cyanoborohydride for coupling). In another exemplary embodiment, heparin may be photolinked on the surface, if it is appropriately grafted with photo initiator moieties. Upon application of this modified heparin formulation on the covalent stent surface, light exposure causes cross-linking and immobilization of the heparin on the coating surface. In yet another exemplary embodiment, heparin may be complexed with hydrophobic quaternary ammonium salts, rendering the molecule soluble in organic solvents (e.g. benzalkonium heparinate, troidodecylmethylammonium heparinate). Such a formulation of heparin may be compatible with the hydrophobic rapamycin coating, and may be applied directly on the coating surface, or in the rapamycin/hydrophobic polymer formulation.

It is important to note that the stent may be formed from any number of materials, including various metals, polymeric materials and ceramic materials. Accordingly, various technologies may be utilized to immobilize the various drug, agent, compound combinations thereon. In addition, the drugs, agents or compounds may be utilized in conjunction with other percutaneously delivered medical devices such as grafts and profusion balloons.

In addition to utilizing an anti-proliferative and anti-coagulant, antiinflammatories may also be utilized in combination therewith. One example of such a combination would be the addition of an anti-inflammatory corticosteroid such as dexamethasone with an anti-proliferative, such as rapamycin, cladribine, vincristine, taxol, or a nitric oxide donor and an anti-coagulant, such as heparin. Such combination therapies might result in a better therapeutic effect, i.e., less proliferation as well as less inflammation, a stimulus for proliferation, than would occur with either agent alone. The delivery of a stent comprising an anti-proliferative, anti-coagulant, and an anti-inflammatory to an injured vessel would provide the added therapeutic benefit of limiting the degree of local smooth muscle cell proliferation, reducing a stimulus for proliferation, i.e., inflammation and reducing the effects of coagulation thus enhancing the restenosis-limiting action of the stent.

In other exemplary embodiments of the inventions, growth factor or cytokine signal transduction inhibitor, such as the ras inhibitor, R115777, or a tyrosine kinase inhibitor, such as tyrphostin, might be combined with an anti-proliferative agent such as taxol, vincristine or rapamycin so that proliferation of smooth muscle cells could be inhibited by different mechanisms. Alternatively, an anti-proliferative agent such as taxol, vincristine or rapamycin could be combined with an inhibitor of extracellular matrix synthesis such as halofuginone. In the above cases, agents acting by different mechanisms could act synergistically to reduce smooth muscle cell proliferation and vascular hyperplasia. This invention is also intended to cover other combinations of two or more such drug agents.

## Claims

1. An intraluminal medical device comprising:
a stent having a substantially tubular body, the tubular body having an inner surface and an outer surface;
a layer of one or more anti-proliferative compounds affixed to the outer surface of the tubular body;
a layer of one or more anti-coagulant compounds affixed to the inner surface of the tubular body; and
a second layer of one or more anti-coagulant compounds affixed to the layer of one or more anti-proliferative compounds affixed to the outer surface of the tubular structure.

2. The intraluminal medical device according to Claim 1, wherein the substantially tubular body comprises a plurality of interconnected bands, each band having an inner surface and an outer surface.

3. The intraluminal medical device according to Claim 2, wherein the layer of one or more anti-proliferative compounds comprises rapamycin.

4. The intraluminal medical device according to Claim 3, wherein the rapamycin is incorporated in a polymeric matrix and immobilized onto the outer surface of the bands.

5. The intraluminal medical device according to Claim 2, wherein the layer of one or more anti-coagulant compounds comprises heparin.

6. The intraluminal medical device according to Claim 5, wherein the heparin is immobilized onto the inner surface of the bands.

7. The intraluminal medical device according to Claim 1, wherein the second layer of one or more anti-coagulant compounds comprises heparin.

8. The intraluminal medical device according to Claim 7, wherein the heparin is immobilized onto the layer of one or more anti-proliferative compounds.

## Patentansprüche

1. Intraluminale medizinische Vorrichtung, umfassend:
einen Stent, der im wesentlichen einen röhrenförmigen Körper aufweist, wobei der röhrenförmige Körper eine innere Oberfläche und eine äußere Oberfläche aufweist;
eine Schicht von einer oder mehreren antiproliferativen Substanzen, aufgebracht auf die äußere Oberfläche des röhrenförmigen Körpers;
eine Schicht von einer oder mehreren anitkoagulanten Substanzen, aufgebracht auf die innere Oberfläche des röhrenförmigen Körpers; und
eine zweite Schicht von einer oder mehreren antikoagulanten Substanzen, aufgebracht auf die Schicht von einer oder mehreren anitproliferativen Substanzen, aufgebracht auf der äußeren Oberfläche der röhrenförmigen Anordnung.

2. Intraluminale medizinische Vorrichtung nach Anspruch 1, wobei der im wesentlichen röhrenförmige Körper eine Vielzahl von zusammenhängenden Bändern umfaßt, wobei jedes Band eine innere Oberfläche und eine äußere Oberfläche aufweist.

3. Intraluminale medizinische Vorrichtung nach Anspruch 2, wobei die Schicht von einer oder mehreren anti-proliferativen Substanzen Rapamycin umfaßt.

4. Intraluminale medizinische Vorrichtung nach Anspruch 3, wobei das Rapamycin in eine Polymermatrix eingeschlossen und auf der äußeren Oberfläche der Bänder immobilisiert ist.

5. Intraluminale medizinische Vorrichtung nach Anspruch 2, wobei die Schicht von einer oder mehreren antikoagulanten Substanzen Heparin umfaßt.

6. Intraluminale medizinische Vorrichtung nach Anspruch 5, wobei das Heparin auf der inneren Oberfläche der Bänder immobilisiert ist.

7. Intraluminale medizinische Vorrichtung nach Anspruch 1, wobei die zweite Schicht von einer oder mehreren antikoagulanten Substanzen Heparin umfaßt.

8. Intraluminale medizinische Vorrichtung nach Anspruch 7, wobei das Heparin auf der Schicht von einer oder mehreren antiproliferativen Substanzen immobilisiert ist.

## Revendications

1. Dispositif médical intraluminal comprenant :
une endoprothèse présentant un corps substantiellement tubulaire, le corps tubulaire ayant une surface interne et une surface externe ;
une couche d'un ou plusieurs composés antiprolifératifs appliqués sur la surface externe du corps tubulaire ;
une couche d'un ou plusieurs composés anticoagulants appliqués à la surface interne du corps tubulaire ; et
une seconde couche d'un ou plusieurs composés anticoagulants appliqués à la couche d'un ou plusieurs composés antiprolifératifs appliqués sur la surface externe de la structure tubulaire.

2. Dispositif médical intraluminal selon la revendication 1, dans lequel le corps substantiellement tubulaire comprend une pluralité de bandes interconnectées, chaque bande ayant une surface interne et une surface externe.

3. Dispositif médical intraluminal selon la revendication 2, dans lequel la couche d'un ou plusieurs composés antiprolifératifs comprend de la rapamycine.

4. Dispositif médical intraluminal selon la revendication 3, dans lequel la rapamycine est incorporée dans une matrice polymérique et immobilisée sur la surface externe des bandes.

5. Dispositif médical intraluminal selon la revendication 2, dans lequel la couche d'un ou plusieurs composés anticoagulants comprend de l'héparine.

6. Dispositif médical intraluminal selon la revendication 5, dans lequel l'héparine est immobilisée sur la surface interne des bandes.

7. Dispositif médical intraluminal selon la revendication 1, dans lequel la seconde couche d'un ou plusieurs composés anticoagulants comprend de l'héparine.

8. Dispositif médical intraluminal selon la revendication 7, dans lequel l'héparine est immobilisée sur la couche d'un ou plusieurs composés antiprolifératifs.
